# EUROPÄISCHE PATENTSCHRIFT

(11) **EP 1 355 593 B1**
(45) Veröffentlichungstag und Bekanntmachung des Hinweises auf die Patenterteilung: **24.05.2006**
(21) Anmeldenummer: 01901107.1
(22) Anmeldetag: 30.01.2001
(51) Int. Cl.: A61F 2/44

(54) **KNOCHENIMPLANTAT, INSBESONDERE ZWISCHENWIRBELIMPLANTAT**
BONE IMPLANT, IN PARTICULAR, AN INTER-VERTEBRAL IMPLANT
IMPLANT OSSEUX, EN PARTICULIER IMPLANT INTERVERTEBRAL

(43) Veröffentlichungstag der Anmeldung: 29.10.2003
(73) Patentinhaber: Synthes AG Chur, 7002 Chur (CH)
(72) Erfinder: MATHYS, Robert, Jr., CH-2544 Bettlach (CH); LECHMANN, Beat, CH-2544 Bettlach (CH); GASSER, Beat, CH-3063 Ittigen (CH)
(74) Vertreter: Lusuardi, Werther
(86) Internationale Anmeldenummer: PCT/CH2001/000069
(87) Internationale Veröffentlichungsnummer: WO 2002/060356

(56) Entgegenhaltungen:
- WO-A-00/66045
- WO-A-97/14377
- WO-A-99/37255
- FR-A- 2 742 653
- US-A- 5 192 327

## Beschreibung

Die Erfindung betrifft ein Knochenimplantat, insbesondere ein Zwischenwirbelimplantat gemäss dem Oberbegriff des Patent anspruchs 1 sowie ein Verfahren zur Herstellung des Knochenimplantats gemäss dem Oberbegriff des Patentanspruchs 16.
Aus der WO00/66045 MICHELSON ist ein derartiges Knochenimplantat bekannt. Die Nachteile dieses bekannten Zwischenwirbelimplantats bestehen darin, dass
A) die glatte Oberfläche der mit makroskopischen Zähnen versehenen Deck- und Grundflächen des Implantats, welche mit den Endplatten der benachbarten Wirbelkörper in Kontakt treten, kein optimales Anwachsen des Knochengewebes gestatten, und dass
B) bei Verwendung eines Füllmaterials im Hohlraum des Implantats, dieses wegen der glatten Innenwände leicht herausfallen kann.

Hier will die Erfindung Abhilfe schaffen. Der Erfindung liegt das Problem zugrunde, ein Knochenimplantat, insbesondere ein Zwischenwirbelimplantat zu schaffen, welches eine für das Anwachsen von Knochengewebe optimale Oberflächenstruktur aufweist.

Die Erfindung löst die gestellte Aufgabe mit einem Knochenimplantat, welches die Merkmale des Anspruchs 1 aufweist und einem Verfahren, welches die Merkmale des Anspruchs 16 aufweist.

Die erfindungsgemässe Oberflächenrauhigkeit von mindestens 2 µm erlaubt dem neu gewachsenen Knochen eine bessere Verankerung am Implantat, d.h. eine bessere Knochenintegration. Die Zwischenwände erhöhen die Auflageflächen auf den benachbarten Endplatten der Wirbelkörper und reduzieren damit die Flächenpressung und verhindern das Einsinken des Implantats in die benachbarten Wirbelkörper. Zudem erlauben die dazwischenliegenden Hohlräume das Durchwachsen des Implantats mit knöcherner Struktur.

Eine Weiterbildung besteht darin, dass die Oberflächenrauhigkeit weniger als 10 µm beträgt. Eine zu grosse Oberflächenrauhigkeit schwächt die verbleibenden Spitzen, was wiederum zu Osteolysen führen kann und damit die Arthrodese beeinträchtigen könnte.
Eine weitere bevorzugte Weiterbildung besteht darin, dass die Zwischenwände mit Perforationen versehen sind, welche vorzugsweise eine Mindestfläche von 3,5 mm² aufweisen. Die Perforationen der Seitenwände dienen der räumlichen Verankerung der neu gewachsenen knöchernen Struktur und auch einer gegebenfalls primär eingebrachten Füllmasse.

Eine weitere bevorzugte Weiterbildung besteht darin, dass die seitliche Abschnitte des Mantels des Hohlkörpers Perforationen aufweisen, welche vorzugsweise eine Mindestfläche von 3,5 mm² aufweisen. Die Perforationen der seitlichen Abschnitte des Mantels dienen der räumlichen Verankerung der neu gewachsenen knöchernen Struktur und primär auch der Implantathalterung während des Einsetzens des Implantates.

Eine weitere bevorzugte Weiterbildung besteht darin, dass das strahlendurchlässigen Material aus folgender Gruppe ausgewählt ist Polyaryletherketon (PAEK), Polyetherimid (PEI), Polyoxy methylen (POM), flüssigkristallines Polymer (LCP), Polymethyl penten (PMP), Polysulfon (PSU), Polyäthersulfon (PESU oder PES), Polyäthylenterephthalat (PETP), Polymethylmethacrylat (PMMA) und ultrahochmolekulares Polyäthylen (UHMW-PE).
Alle diese bevorzugten Materialien sind elastisch, verfügen aber über unterschiedliche, mechanische Eigenschaften, wie Elastizität (Steifigkeit) und auch Festigkeit. Im Vergleich zu anderen Polymeren weisen sie zum Teil günstige Kriecheigenschaften aus oder zeigen einer geringe Wasseraufnahme. Damit ist je nach Anwendungsbedarf eine optimale Auswahl möglich.

Eine weitere bevorzugte Weiterbildung besteht darin, dass das strahlendurchlässigen Material faserverstärkt ist, vorzugsweise mit Kohlefasern oder mit PEEK-Fasern. Eine Faserverstärkung des strahlendurchlässigen Materials versteift die mechanischen Eigenschaften im allgemeinen oder gezielt.

Eine weitere bevorzugte Weiterbildung besteht darin, dass das Verhältnis V:v zwischen Gesamtvolumen V des Knochenimplantats und dem Volumen v des Hohlkörpers im Bereich von 1,9 bis 2,3 liegt. Es hat sich gezeigt, dass dieses Verhältnis die Vorteile eines mechanisch stabilen Implantats mit einem maximalen Volumen von neu gewachsener, knöcherner Struktur vereinigt.

Eine weitere bevorzugte Weiterbildung besteht darin, dass die Oberfläche des Implantats mindestens teilweise mit einer röntgenstrahlendurchlässigen Beschichtung oder einer dünnen, die Röntgenstrahlendurchlässigkeit nicht beeinflussenden Beschichtung versehen ist. Der Vorteil der röntgentransparenten Beschichtung sichert die Beobachtungsmöglichkeiten während des Heranwachsens der Arthrodese. Während sich reines PEEK bereits durch eine hervorragende Biokompatibilität auszeichnet, steigert eine zusätzliche Beschichtung aus einem geeigneten Werkstoff die mechanischen Eigenschaften und die Interfaces zwischen dem neu gewachsenen Knochen und dem Implantat. Als Beschichtungsmaterial eigenen sich vorzugsweise Metalle (in einer sehr geringen Dicke, welche die Röntgenstrahlendurchlässigkeit nicht wesentliche beeinträchtigt. Insbesondere eignen sich Titan, Gold oder Platin oder andere geeignete Implantatmetalle.

Eine weitere bevorzugte Weiterbildung besteht darin, dass die Beschichtung aus einem keramischen Stoff besteht, vorzugsweise aus Hydroxylapatit oder Tricalciumphosphat. Beide Keramiken, Hydroxylapatit und Tricalciumphosphat, die sich für eine Beschichtung eigenen, zeichnen sich durch den Vorteil aus, dass sie vollständig in den Knochen integriert werden oder sogar durch neues, natürliches Knochengewebe ersetzt werden.

Eine weitere bevorzugte Weiterbildung besteht darin, dass der Hohlkörper mindestens teilweise mit einem Füllmittel aus Calciumphosphat, vorzugsweise Hydroxylapatit oder Tricalciumphosphat gefüllt ist. Damit wird eine bessere, störungsfreiere Kontrolle der Arthrodese erzielt. Der Hohlkörper kann auch mit einem Füllmittel aus Calciumsulfat, demineralisiertem Knochen, autologem Knochen oder korallinen Substanzen gefüllt werden.

Eine weitere bevorzugte Weiterbildung besteht darin, dass der Hohlkörper (1) mindestens teilweise mit einem röntgenopaken Füllmittel aus einem resorbierbaren, vorzugsweise porösen Polymer gefüllt ist. Um das Füllmittel röntgenopak zu gestalten, werden dem Polymer Kontrastmittel zugesetzt. Bei PLA kann man z.B. einen Marker, wie z.B. Zirkondioxid zusetzen, zu PEEK kann Bariumsulfat beigemischt werden.

Eine weitere bevorzugte Weiterbildung besteht darin, dass die gegebenfalls vorhandenen Perforationen im Implantat mindestens teilweise mit dem röntgenopaken Füllmittel gefüllt sind. Dadurch wird das Füllmittel am Herausfallen gehindert.

Das Verfahren zur Herstellung eines erfindungsgemässen Knochenimplantats besteht darin, dass der ringförmige Hohlkörper durch Spritzgiess-, Warmform-, oder Warmpress-Technik hergestellt wird. Durch die Warmverformung wird das Polymer in allen Formelementen verdichtet, was sich insbesondere durch eine bessere Ermüdungsfestigkeit auszeichnet.

Die Erfindung und Weiterbildungen der Erfindung werden im folgenden anhand der teilweise schematischen Darstellung eines Ausführungsbeispiels noch näher erläutert.

Es zeigen:
Fig. 1 die perspektivische Darstellung eines erfindungs gemässen Zwischenwirbelimplantats;
Fig. 2 eine Aufsicht auf das Implantat gemäss Fig. 1;
Fig. 3 eine Vorderansicht des Implantats gemäss Fig. 1; und
Fig. 4 eine Seitenansicht des Implantats gemäss Fig. 1.

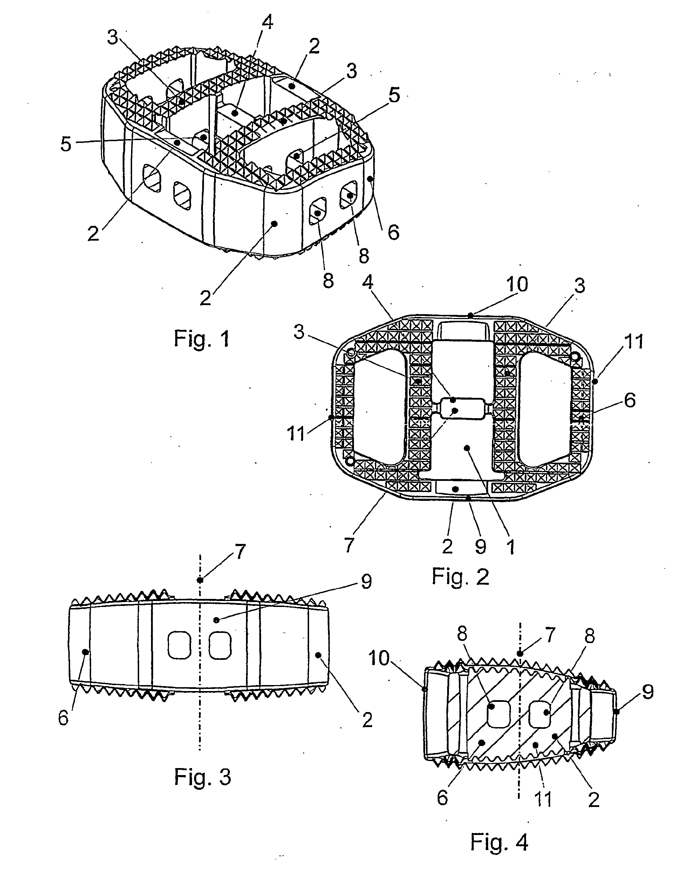

Das in den Figuren 1 bis 4 dargestellte Zwischenwirbelimplantat besteht aus einem strahlendurchlässigen Material (PEEK, welches wahlweise mit Kohlefasern oder Glasfasern verstärkt sein kann), welches im Spritzgussverfahren als ringförmiger Hohlkörper 1 ausgebildet ist. Es umfasst einen hohlzylinder abschnittförmigen Mantel 6, der einen vorderen Abschnitt 9 einen hinteren Abschnitt 10 und zwei seitliche Abschnitte 11 aufweist sowie eine Zylinderachse 7 definiert.
Der Hohlkörper 1 ist durch zwei Zwischenwände 3, welche im wesentlichen parallel zur Zylinderachse 7 verlaufen und den vorderen Abschnitt 9 mit dem hinteren Abschnitt 10 des Mantels verbindet, in Kammern unterteilt. Die beiden Zwischenwände 3 sind mittels einer Querstrebe 4 miteinander verstrebt. Die Zwischenwände 3 sind mit Perforationen 5 versehen, welche eine Fläche von ca. 10 mm² aufweisen. Auch die seitliche Abschnitte 11 des Mantels 6 weisen Perforationen 8 auf, welche eine Fläche von ca. 10 mm² aufweisen.

Die Oberfläche 2 des Knochenimplantats weist eine Oberflächen rauhigkeit von 6 µm auf und ist mit einer dünnen, röntgenstrahlendurchlässigen Beschichtung aus Gold versehen.

Das Verhältnis V:v zwischen Gesamtvolumen V des Knochenimplantats und dem Volumen v des Hohlkörpers 1 beträgt ca. 2,1.

Der Hohlkörper 1 kann mit einem röntgenopaken Füllmittel aus Hydroxylapatit aufgefüllt sein, wobei darauf zu achten ist, dass vorzugsweise auch die vorhandenen Perforationen 5;8 mit dem röntgenopaken Füllmittel gefüllt sind.

## Patentansprüche

1. Knochenimplantat, insbesondere Zwischenwirbelimplantat, welches aus einem röntgen strahlendurchlässigen Material besteht und als ringförmiger Hohlkörper (1) ausgebildet ist mit einem hohlzylinderabschnittförmigen Mantel (6), der einen vorderen (9), einen hinteren (10) und zwei seitliche Abschnitte (11) umfasst sowie eine Zylinderachse (7) definiert, wobei der Hohlkörper (1) durch mindestens zwei Zwischenwände (3) unterteilt ist, welche im wesentlichen parallel zur Zylinderachse (7) verlaufen und den vorderen Abschnitt (9) mit dem hinteren Abschnitt (10) des Mantels verbinden,
**dadurch gekennzeichnet, dass**
A) die Oberfläche (2) des Knochenimplantats eine Oberflächenrauhigkeit von mindestens 2 µm aufweist; und
B) die Zwischenwände (3) mittels einer Querstrebe (4) miteinander verstrebt sind.

2. Knochenimplantat nach Anspruch 1, **dadurch gekennzeichnet, dass** die Oberflächenrauhigkeit mindestens zum und weniger als 10 µm beträgt.

3. Knochenimplantat nach Anspruch 1 oder 2, **dadurch gekennzeichnet, dass** die Zwischenwände (3) mit Perforationen (5) versehen sind.

4. Knochenimplantat nach Anspruch 3, **dadurchgekennzeichnet**, dass die Perforationen (5) eine Mindestfläche von 3,5 mm² aufweisen.

5. Knochenimplantat nach einem der Ansprüche 1 bis 4, **dadurch gekennzeichnet, dass** die seitliche Abschnitte (11) des Mantels (6) Perforationen (8) aufweisen, welche vorzugsweise eine Mindestfläche von 3,5 mm² aufweisen.

6. Knochenimplantat nach einem der Ansprüche 1 bis 5, **dadurch gekennzeichnet, dass** das strahlendurchlässige Material aus folgender Gruppe ausgewählt ist: Polyaryletherketon (PAEK), Polyetherimid (PEI), Polyoxymethylen (POM), flüssigkristallines Polymer (LCP), Polymethylpenten (PMP), Polysulfon (PSU), Poly äthersulfon (PESU oder PES), Polyäthylenterephthalat (PETP), Polymethylmethacrylat (PMMA) und ultrahochmolekulares Polyäthylen (UHMW-PE).

7. Knochenimplantat nach einem der Ansprüche 1 bis 6, **dadurch gekennzeichnet, dass** das strahlendurchlässigen Material faserverstärkt ist, vorzugsweise mit Kohlefasern, Glasfasern oder PEEK-Fasem.

8. Knochenimplantat nach einem der Ansprüche 1 bis 7, **dadurch gekennzeichnet, dass** das Verhältnis V:v zwischen Gesamtvolumen V des Knochenimplantats und dem Volumen v des Hohlkörpers (1) im Bereich von 1,9 bis 2,3 liegt.

9. Knochenimplantat nach einem der Ansprüche 1 bis 8, **dadurch gekennzeichnet, dass** seine Oberfläche mindestens teilweise mit einer röntgenstrahlendurchlässigen Beschichtung oder einer dünnen, die Röntgenstrahlendurchlässigkeit wenig beeinflussenden Beschichtung versehen ist.

10. Knochenimplantat nach Anspruch 9, **dadurch gekennzeichnet, dass** die Beschichtung aus einem Metall besteht, vorzugsweise aus Titan, Gold oder Platin.

11. Knochenimplantat nach Anspruch 9, **dadurch gekennzeichnet, dass** die Beschichtung aus einem keramischen Stoff besteht, vorzugsweise aus Hydroxylapatit oder Tricalciumphosphat.

12. Knochenimplantat nach einem der Ansprüche 1 bis 11, **dadurch gekennzeichnet, dass** der Hohlkörper (1) mindestens teilweise mit einem Füllmittel aus Calciumphosphat, vorzugsweise Hydroxylapatit oder Tricalciumphosphat gefüllt ist.

13. Knochenimplantat nach einem der Ansprüche 1 bis 11, **dadurch gekennzeichnet, dass** der Hohlkörper (1) mindestens teilweise mit einem Füllmittel aus Calciumsulfat, demineralisiertem Knochen, autologem Knochen oder korallinen Substanzen gefüllt ist.

14. Knochenimplantat nach einem der Ansprüche 1 bis 11, **dadurch gekennzeichnet, dass** der Hohlkörper (1) mindestens teilweise mit einem Füllmittel aus einem resorbierbaren, vorzugsweise porösen Polymer gefüllt ist.

15. Knochenimplantat nach einem der Ansprüche 4 bis 14, **dadurch gekennzeichnet, dass** die gegebenfalls vorhandenen Perforationen (5;8) mindestens teilweise mit dem röntgenopaken Füllmittel gefüllt sind.

16. Verfahren zur Herstellung eines Knochenimplantats gemäss einem der Ansprüche 1 bis 15, **dadurch gekennzeichnet, dass** der ringförmige Hohlkörper (1) durch Spritzgiess-, Warmform-, oder Warmpress-Technik hergestellt wird.

## Claims

1. A bone implant, in particular an intervertebral implant which consists of a material being radiolucent for X-rays and is shaped in the form of an annular, hollow body (1) including a lateral area (6) in the form of a segment of a hollow cylinder which comprises a front segment (9), a rear segment (10), as well as two lateral segments (11), and which defines a cylinder axis (7), whereby the hollow body (1) being subdivided by at least two partitions (3) extending substantially parallel to the cylinder axis (7) and connecting the front segment (9) with the rear segment (10) of the lateral area,
**characterised in that**
A) the surface (2) of the bone implant has a surface roughness of at least 2 µm; and
B) said partitions (3) being braced against one another by means of a transverse brace (4).

2. A bone implant as claimed in claim 1, **characterised in that** the surface roughness is at least 2 µm and inferior to 10 µm.

3. A bone implant as claimed in claim 1 or 2, **characterised in that** the partitions (3) are provided with perforations (5).

4. A bone implant as claimed in any of the claims 1 to 3, **characterised in that** the perforations in the partitions (3) have a minimum surface of 3.5 mm².

5. A bone implant as claimed in any of the claims 1 to 4, **characterised in that** the lateral segments (11) of the lateral area (6) are provided with perforations (8) which preferably have a minimum surface of 3.5 mm².

6. A bone implant as claimed in any of the claims 1 to 5, **characterised in that** the radiolucent material is selected from the following group: polyaryletherketone (PAEK), polyetherimide (PEI), polyoxymethylene (POM), liquid crystal polymer (LCP), polymethylpentene (PMP), poiysuifone (PSU), polyethersulfone (PESU or PES), polyethyleneterephthalate (PETP), polymethylmethacrylate (PMMA), and ultra-high molecular weight polyethylene (UHMWPE).

7. A bone implant as claimed in any of the claims 1 to 6, **characterised in that** the radiolucent material is fibre reinforced, the reinforcement material being preferably carbon fibre or PEEK fibre.

8. A bone implant as claimed in any of the claims 1 to 7, **characterised in that** the ratio V:v between the total volume V of the bone implant and the volume v of the hollow body (1) is in a range of between 1.9 and 2.3.

9. A bone implant as claimed in any of the claims 1 to 8, **characterised in that** its surface is at least partially provided with a coating radiolucent for X-rays or with a thin coating which does not substantially affect the radiolucency for X-rays.

10. A bone implant as claimed in claim 9, **characterised in that** the coating consists of a metal, preferably of titanium, gold or platinum.

11. A bone implant as claimed in claim 9, **characterised in that** the coating consists of a ceramic material, preferably of hydroxyapatite or tribasic calcium phosphate.

12. A bone implant as claimed in any of the claims 1 to 11, **characterised in that** the hollow body (1) is at least partially filled with a filling material consisting of calcium phosphate, preferably of hydroxyapatite or tribasic calcium phosphate.

13. A bone implant as claimed in any of the claims 1 to 11, **characterised in that** the hollow body (1) is filled with a filling material consisting of calcium suiphate, demineralised bone, autologous bone or coralline substances.

14. A bone implant as claimed in any of the claims 1 to 11, **characterised in that** the hollow body (1) is at least partially filled with a filling material consisting of a resorbable, preferably porous polymer.

15. A bone implant as claimed in any of the claims 4 to 14, **characterised in that** the perforations (5;8) which may be present in the implant are at least partially filled with the radiopaque filling material.

16. A method for fabricating a bone implant as claimed in any of the claims 1 to 15, **characterised in that** the annular, hollow body (1) is fabricated by means of an injection moulding, a hot forming or a hot pressing technique.

## Revendications

1. Implant d'ostéosynthèse, notamment implant intervertébral, qui est constitué par un matériau transparent aux rayons X et qui est formé en tant que corps creux (1) de forme annulaire, comportant une enveloppe (6) en forme de segment de cylindre creux, laquelle comprend une partie avant (9), une partie arrière (10) et deux parties latérales (11) et qui définit en outre un axe de cylindre (7), le corps creux (1) étant subdivisé par au moins deux parois intermédiaires (3) qui s'étendent essentiellement parallèlement à l'axe de cylindre (7) et qui relient la partie avant (9) à la partie arrière (10) de l'enveloppe,
**caractérisé en ce que**
A) la surface (2) de l'implant d'ostéosynthèse présente une rugosité de surface d'au moins 2 µm; et
B) les parois intermédiaires (3) sont reliées entre elles au moyen d'une entretoise (4).

2. Implant d'ostéosynthèse selon la revendication 1, **caractérisé en ce que** la rugosité de surface est d'au moins 2 µm et fait moins de 10 µm.

3. Implant d'ostéosynthèse selon la revendication 1 ou 2, **caractérisé en ce que** les parois intermédiaires (3) sont pourvues de perforations (5).

4. Implant d'ostéosynthèse selon la revendication 3, **caractérisé en ce que** les perforations (5) présentent une surface minimum de 3,5 mm².

5. Implant d'ostéosynthèse selon l'une des revendications 1 à 4, **caractérisé en ce que** les parties latérales (11) de l'enveloppe (6) sont pourvues de perforations (8) qui présentent de préférence une surface minimum de 3,5 mm².

6. lmplant d'ostéosynthèse selon l'une des revendications 1 à 5, **caractérisé en ce que** le matériau transparent au rayonnement est sélectionné parmi le groupe suivant :
cétone de polyéther (PAEK), polyétherimide (PEI), polyoxyméthylène (POM), polymère de cristal liquide (LCP), polyméthylpentène (PMP), polysulfone (PSU), polyéthersulfone (PESU ou PES), téréphthalate de polyéthylène (PETP), polyméthylméthacrylate (PMMA) et polyéthylène à ultra-haute densité moléculaire (UHMW-PE).

7. Implant d'ostéosynthèse selon l'une des revendications 1 à 6, **caractérisé en ce que** le matériau transparent au rayonnement est renforcé par fibres, de préférence par des fibres de carbone, des fibres de verre ou des fibres de PEEK.

8. Implant d'ostéosynthèse selon l'une des revendications 1 à 7 **caractérisé en ce que** le rapport V:v entre le volume total V de l'implant d'ostéosynthèse et le volume v du corps creux (1) se situe dans une plage comprise entre 1,9 et 2,3.

9. Implant d'ostéosynthèse selon l'une des revendications 1 à 8, **caractérisé en ce que** sa surface est pourvue, au moins partiellement, d'un revêtement transparent au rayons X ou d'un revêtement de faible épaisseur influençant peu la transparence aux rayons X.

10. Implant d'ostéosynthèse selon la revendication 9, **caractérisé en ce que** le revêtement est constitué par un métal, de préférence par du titane, de l'or ou du platine.

11. Implant d'ostéosynthèse selon la revendication 9 **caractérisé en ce que** le revêtement est constitué par une matière céramique, de préférence par de l'hydroxylapatite ou du phosphate tricalcique.

12. Implant d'ostéosynthèse selon !'une des revendications 1 à 11 **caractérisé en ce que** le corps creux (1) est rempli, au moins partiellement, d'une matière de remplissage constituée par du phosphate de calcium, de préférence de l'hydroxylapatite ou du phosphate tricalcique.

13. Implant d'ostéosynthèse selon l'une des revendications 1 à 11, **caractérisé en ce que** le corps creux (1) est rempli, au moins partiellement, d'une matière de remplissage constituée par du sulfate de calcium, de l'os déminéralisé, de l'os autologue ou des substances coralliennes.

14. Implant d'ostéosynthèse selon l'une des revendications 1 à 11, **caractérisé en ce que** le corps creux (1) est rempli, au moins partiellement, d'une matière de remplissage constituée par un polymère résorbable, de préférence poreux.

15. Implant d'ostéosynthèse selon l'une des revendications 4 à 14, **caractérisé en ce que** les perforations (5;8) existantes sont remplies le cas échéant, au moins partiellement, avec la matière de remplissage opaque aux rayons X.

16. Procédé permettant la fabrication d'un implant d'ostéosynthèse selon l'une des revendications 1 à 15, **caractérisé en ce que** le corps creux (1) de forme annulaire est fabriqué par technique de moulage par injection, de thermoformage ou d'estampage à chaud.
